# EUROPÄISCHE PATENTSCHRIFT

(11) **EP 0 367 811 B1**
(45) Veröffentlichungstag und Bekanntmachung des Hinweises auf die Patenterteilung: **04.08.1993**
(21) Anmeldenummer: 89904544.7
(22) Anmeldetag: 07.04.1989
(51) Int. Cl.: A61B 5/00

(54) **VERFAHREN UND VORRICHTUNG ZUR DIAGNOSE BEI POLYNEUROPATHIESYNDROMEN**
PROCESS AND DEVICE FOR DIAGNOSIS IN THE CASE OF POLYNEUROPATHIC SYNDROMES
PROCEDE ET DISPOSITIF DE DIAGNOSTIQUE POUR LES SYNDROMES POLYNEUROPATHIQUES

(30) Priorität: 08.04.1988 DE 3811855; 22.09.1988 DE 3832245
(43) Veröffentlichungstag der Anmeldung: 16.05.1990
(73) Patentinhaber: Költringer, Peter, Dr., A-8041 Graz (AT)
(72) Erfinder: Költringer, Peter, Dr., A-8041 Graz (AT)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)
(86) Internationale Anmeldenummer: EP8900378
(87) Internationale Veröffentlichungsnummer: WO8909562

(56) Entgegenhaltungen:
- EP-A- 0 019 444
- EP-A- 0 157 962
- US-A- 4 590 948
- Medical & Biological Engineering & Computing volume 25, No. 3, May 1987, IFMBE, (Stevenage, Herts, GB), Z.-G. Qiao et al.: Simultaneous measurement of electrical admittance, blood flow and temperature at the same skin site with a specially designed probe", pages 299-304

## Beschreibung

Die vorliegende Erfindung betrifft ein Verfahren bzw. eine Vorrichtung zur Feststellung, ob bei einem Probanden eine Nervenschädigung (Polyneuropathie) vorhanden ist bzw. des Grades der Nervenschädigung.

Bei zahlreichen Erkrankungen treten Polyneuropathiesyndrome (PNS) als häufige Komplikation auf. Allen voran ist hier der Diabetes mellitus zu nennen. In einer ausführlichen Untersuchung von Canal et al. (Canal N, Pozza G(eds): Peripheral neuropathies. Elsevier North-Holland, Amsterdam, S. 247-255) wurde festgestellt, daß nach einem Verlauf von mehr als 5 Jahren eines insulinpflichtigen Diabetes mellitus lediglich 18% noch nicht an einem manifesten Polyneuropathiesyndrom erkrankt sind. Als weitere häufige Ursachen kommen vor allem auch andere Stoffwechselstörungen wie z.B im Rahmen eines chronischen Äthylismus bei verschiedenen Vergiftungen oder auch neoplastische und entzündliche Prozesse in Betracht.

Die Klinik des PNS ist sehr unterschiedlich und zahlreiche Einteilungen wurden in den letzten Jahren vorgeschlagen (Brown MJ, Asbury AK (1984) Diabetic neuropathy. Ann.Neurol 15: 2 - 12). Der übliche Verlauf zeigt meist einen Beginn an den unteren Extremitäten in Form von schmerzhaften Mißempfindungen, die anfangs vor allem nachts auftreten. In der weiteren Folge sind socken- und handschuhförmige Sensibilitätsstörungen, Verlust der Tiefensensibilität, Areflexie beginnend bei Achillessehnenreflex und schließlich Paresen zu nennen, von welchen die häufigste die Peronäusparese darstellt.

Aufgrund dieser Vielfalt an Symptomen, die meist kombiniert mit unterschiedlicher Dominanz auftreten, wurden auch zahlreiche Versuche zur Objektivierung des PNS in den vergangenen 3 Jahrzehnten unternommen, siehe z.B. Hoffmann A, Conen D, Leibundgut U, Berger W (1982) A skin test for autonomic neuropathy. Eur Neurol 21: 29 - 33; Kennedy WR, Sakuda M. Sutherland D. Goetz FC (1984) The sweating deficiency in diabetes mellitus: methods of guantification and clinical correlation. Neurology 34: 758 - 763; und Ward JD, Fisher DJ, Barnes CG, Jessop JD (1971) Improvement in nerve conduction following treatment in newly diagnosed diabetes. Lancet i: 428). Als eine der wenigen routinemäßig durchführbaren Untersuchungen blieb die Messung der motorischen Nervenleitgeschwindigkeit von Ward et al, welche in der klinischen Routine am Nervus peronaeus erfolgt, da dieser am häufigsten betroffen ist. Diese Diagnostik zeigt jedoch meist erst dann Veränderungen, wenn bereits schwerere Ausfälle vorhanden sind. Gerade aber in diesem Stadium sind die Therapiemögliohkeiten nicht mehr zufriedenstellend.

Die Aufgabe der vorliegenden Erfindung ist es daher, ein diagnostisches Verfahren bzw. eine Vorrichtung zur Durchführung dieses Verfahrens vorzuschlagen, das bzw. die die frühzeitige Erkennung von neuropathischen oder polyneuropathischen Veränderungen ermöglicht, im Routinebetrieb durchführbar bzw. verwendbar ist und somit eine rechtzeitige therapeutische Behandlung der Neuropathie ermöglicht. Weiterhin soll das Verfahren bzw. die Vorrichtung eine objektive Beurteilung des vom Patienten empfundenen Schmerzes ermöglichen.

Zur Lösung dieser Aufgabe wird erfindungsgemäß zunächst das Verfahren nach Anspruch 1 bzw. die Vorrichtung nach Anspruch 6 vorgeschlagen.

Die vorliegende Erfindung geht von der Überlegung aus, daß die autonomen Nervenfasern bei polyneuropathischen Veränderungen meist sehr früh mitbefallen sind, wobei der Zustand der autonomen Nervenfasern über eine mögliche Dysfunktion der Haut erfaßbar sein könnte. Danach wurde spekuliert, daß eine solche Dysfunktion der Haut durch eine Veränderung der Mikrozirkulation eventuell feststellbar wäre.

Nach Durchführung von Mikrozirkulationsmessungen bei mehreren Probanden mit unterschiedlichen Krankheitsstadien der Polyneuropathie wurde aber festgestellt, daß die ermittelten Perfusionswerte einer sehr großen Streuung unterlagen und daß keine verwertbare Korrelation mit dem Grad der Krankheit vorlag. Es wurde dann aber überraschenderweise festgestellt, daß bei Erwärmung der Haut der einzelnen Probanden, um eine Zunahme der Mikrozirkulation zu provozieren, die Zeit vom Einsetzen der Hauttemperaturzunahme bis zum Einsetzen der Perfusionszunahme aufgrund der erhöhten Hauttemperatur eine sehr zuverlässige Korrelation mit dem mit anderen Methoden ermittelten Krankheitszustand aufweist. Die gemessene Zeit, Hyperthermielatenz bezeichnet, bildet somit eine zuverlässige Meßmethode zur Feststellung, ob eine Nervenschädigung vorliegt bzw. welchen Grad sie aufweist. Die Hyperthermielatenz wird umso größer, je schwerer die Nervenschädigungen an den Gefäßen der Extremität sind (Bestandteil des sog. "autonomen Nervensystems"). Es wird sogar festgestellt, daß bei schweren Polyneuropathiesyndromen überhaupt kein Anstieg der Perfusion mehr feststellbar ist. Die Hyperthermielatenz ist dort daher auch nicht meßbar, da die Perfusion am Ausgangsniveau bleibt. Auch dieses Ergebnis ist jedoch von Bedeutung, da man hierdurch eine Bestätigung hat, daß eine Nervenschädigung schwersten Grades vorliegt.

Besonders bevorzugte Ausführungsformen des erfindungsgemäßen Verfahrens bzw. der erfindungsgemäßen Vorrichtung sind den abhängigen Ansprüchen zu entnehmen.

Besonders vorteilhaft ist vor allem die Vorrichtung nach den Ansprüchen 13 und 14, wonach ein Computer zur Erstellung und Auswertung der Hyperthermielatenz und zur Erstellung eines Protokolls über die durchgeführte Messung verwendet wird. Hiermit besteht die Möglichkeit, die Messungen mit relativ ungeübtem Personal durchzuführen und langfristig den Zustand eines bestimmten Patienten zu beobachten und den Verlauf seines Krankheitsbildes bzw. der therapeutischen Erfolge zu ermitteln durch einen Vergleich der an verschiedenen Tagen verteilt über einen längeren Zeitraum ermittelten Protokolle. Auch diese langfristige Auswertung kann vom Computer vorgenommen werden.

Besonders vorteilhaft bei dem erfindungsgemäßen Verfahren bzw. Vorrichtung ist, daß die Messung schnell und für den Patienten schmerzfrei durchgeführt werden kann, wobei die Ergebnisse äußerst zuverlässig sind und bereits im frühen Stadium einer Polyneuropathie ein zuverlässiges Ergebnis liefern, so daß frühzeitig mit der therapeutischen Behandlung der Polyneuropathie begonnen werden kann.

Eine verwandte, jedoch alternative Lösung der eingangs gestellten Aufgabe ist verfahrensmäßig durch den Anspruch 15 und vorrichtungsmäßig durch den Anspruch 18 gekennzeichnet.

Diese alternative Lösung basiert auf der Erkenntnis, daß die Temperaturzunahme der Haut im Bereich der beheizten Extremität jedoch an einer Stelle, die nichtdirekt beheizt wird, mit der der Perfusionswerte an bzw. nahe dieser Stelle korreliert ist, da eine Zunahme der Perfusion einer Zunahme des Wärmetransports durch das Blut und damit auch einer Temperaturänderung gleichzusetzen ist.

Eine statistische Analyse von an Probanden gemessenen Werten hat die Korrelation eindeutig nachgewiesen.

Vorteilhafte Weiterbildungen dieser alternativen Lösung sind den abhängigen Ansprüchen, 16, 17 und 19 zu entnehmen.

Die Erfindung wird nachfolgend aufgrund einer Beschreibung eines Beispiels der erfindungsgemäßen Vorrichtung sowie einer Beschreibung einer klinischen Untersuchung unter Bezugnahme auf die beigefügte Zeichnung näher erläutert. In der Zeichnung zeigt:
- Fig. 1: eine schematische Darstellung der erfindungsgemäßen Vorrichtung,
- Fig. 2: eine graphische Darstellung des Ausdruckes des Computers der Fig. 1,
- Fig. 3a bis 3c: tabellarisch Ergebnisse der klinischen Untersuchung von 40 Probanden, und zwar im Hinblick auf die gemessenen Hyperthermielatenz, Ausgangsperfusionswerte und Maximalperfusionswerte zusammen mit ihren Signifikanzen im Wilcoxon-Test sowie in Klammer stehend im Chi-Square-Test,
- Fig. 4a bis 4c: graphische Darstellungen der prozentuellen Medianunterschiede der drei Parameter Hyperthermielatenz, Ausgangsperfusionswerte und Maximalperfusionswerte, die bei der Untersuchung festgestellt wurden, und
- Fig. 5: eine schematische Darstellung einer alternativen erfindungsgemäßen Vorrichtung.

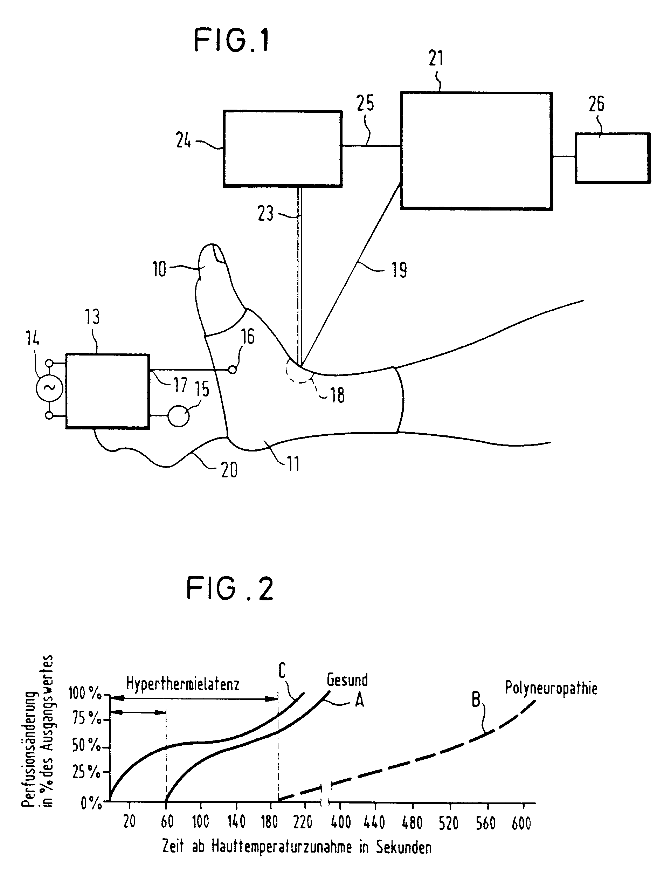

Die Fig. 1 zeigt eine Extremität eines Probanden in Form seines rechten Fußes 10, der mit einer Heizdecke 11 im Knöchelbereich abgedeckt ist. Die Heizdecke 11 wird um den Fuß geschlagen, es könnte aber auch eine einfache Decke oder ein Heizkissen verwendet werden. Die Heizdecke ist elektrisch beheizt, wobei die elektrische Energie zur Beheizung der Heizdecke über eine Regelschaltung 13 und eine Leitung 20 der Heizdecke zugeführt wird. Die Regelschaltung 13 ist am Netz 14 angeschlossen und hat einen Sollwerteingang 15, dem ein Referenzwert entsprechend der erwünschten Temperatur der Heizdecke 11, im vorliegenden Beispiel 44°, zugeführt wird. Die eigentliche Temperatur der Heizdecke ist, von einem Temperaturfühler 16 ermittelt, an einen Istwerteingang 17 der Regelschaltung 13 angelegt. Eine Temperatursonde 18 zur Ermittlung der Hauttemperatur des Patienten ist direkt an der Haut des Probanden angebracht unterhalb der Heizdecke, dort wo sie zugeschlagen wird. Die von dieser Temperatursonde 18 ermittelte Temperatur wird über eine Leitung 19 an einen Auswertungscomputer 21 angelegt. Die Temperatursonde hat einen Meßkopf mit einem Durchmesser von etwa 3 cm und in der Mitte ein kleines Loch. Dort wird die Lasersonde 23 eines bekannten Laserperfusionsmeßgeräts 24 plaziert. Die grundsätzliche Methodik der Laserperfusionsmessung ist in zahlreichen Untersuchungen beschrieben (siehe z.B. Shepherd AP, Riedel GL (1982) Continuous measurement of intestinal mucosal bloodflow by Laser-Doppler Velocimetry. Am J Physiol 242: G669 - G672; Stern MD, Lappe DL, Bown PD, Chimosky JE, Holloway GA, Keiser HR (1977) Continuous measurement of tissue blood flow by Laser-Doppler-Spectroscopy. Am J Physiol 232: H441 - H448; und Svensson H, Svedman P, Holmberg J, Wieslander JB (1983). Continuous monitoring of circulation in flaps. Transactions of the VIII International Congress of plastic surgery, Montreal, June 26 - July 1). Das Prinzip dieser Untersuchung beruht auf einem Doppler-Effekt, der einerseits durch den Kapillarhämatokrit und andererseits durch die mittlere Strömungsgeschwindigkeit der Teilchen im Kapillargebiet beeinflußt wird. Über eine Computerauswertung werden diese zwei bestimmenden Parameter zur "Perfusionseinheit" umgerechnet. Der hierfür zuständige Computer ist im Laserperfusionsmeßgerät 24 untergebracht. Im vorliegenden Fall wurde zur Durchführung der Perfusionsmessung ein PeriFlux PF3 Gerät der Firma Perimed in Schweden benutzt. Dieses Gerät dürfte in der US-PS 4 590 948 beschrieben sein. Die Temperatursonde ist ein Bestandteil des PeriFlux-Gerätes und wird normalerweise dazu benutzt, die Temperatur an der Meßstelle zu erfassen und über eine Heizeinrichtung und eine Regelschaltung konstant zu halten, um Meßverfälsohungen zu vermeiden. Im vorliegenden Fall wird die Sonde lediglich zur Messung der Temperatur benutzt.

Die vom Laserperfusionsmeßgerät 24 ermittelten Meßwerte "Perfusionseinheiten" werden über die Leitung 25 ebenfalls dem Computer 21 zugeführt.

Der Computer erstellt für jeden Probanden ein Protokoll in Form einer Computergraphik über die ermittelten Änderungen der Perfusionswerte in Form eines Prozentsatzes des Ausgangswertes als Funktion der Zeit in Sekunden ab einer Hauttemperaturzunahme des Probanden aufgrund der Wirkung der Heizdecke. Diese Computergraphik wird über einen Drucker 26 ausgedruckt. Die Hauttemperaturzunahme wird aufgrund einer Mittelwertbildung vom Computer errechnet. Die für die vorliegende Erfindung wichtige Zeit, die sog. Hyperthermielatenz ist die Zeit vom Einsetzen der Hauttemperaturzunahme, die durch die Heizdecke am Fuß verursacht wird, bis zum Einsetzen der Perfusionszunahme aufgrund der erhöhten Hauttemperatur. Auch die Perfusionszunahme wird vom Computer durch Mittelwertbildung festgestellt, wobei nur die Werte ab der festgestellten Zunahme in der Graphik dargestellt werden, um das Bild für den behandelnden Arzt zu vereinfachen. Das Protokoll gemäß Fig. 2 zeigt eine Kurve A, die für gesunde Patienten gilt, und als Kurve C die gemessene Hauttemperaturzunahme. Alle Patienten, bei denen Kurven ermittelt werden, die auf der linken Seite der Kurve A liegen, können als gesund eingestuft werden. Liegt die für einen bestimmten Patienten ermittelte Kurve jedoch auf der rechten Seite von der Kurve A, z.B. die Kurve B, so leidet dieser Patient unter einer Polyneuropathie. Der Grad der Polyneuropathie läßt sich aufgrund der ermittelten Hyperthermielatenz feststellen. Bei dem vorliegenden Beispiel liegt die Hyperthermielatenz bei 190 Sekunden im Vergleich zu einem Wert von weniger als 60 Sekunden bei einem gesunden Patienten. Bei sehr schweren Polyneuropathiesyndromen ist überhaupt kein Anstieg der Perfusion mehr feststellbar, die Hyperthermielatenz ist dort daher auch nicht meßbar, da die Perfusion am Ausgangsniveau bleibt. Die Feststellung, daß die Perfusion nicht steigt, ist für sich eine Bestätigung, daß bereits ein Polyneuropathiesyndrom schwersten Grades vorliegt.

Der Ablauf einer Messung ist wie folgt. Zunächst wird das Aufwärmen der Heizdecke auf 44°C durch Schließung eines Schalters (nicht gezeigt) gestartet. Parallel dazu startet die Laser-Doppler-Flowmetriemessung. Die Messung wird über einen Zeitraum von bis zu 10 Minuten durchgeführt und es werden Hauttemperaturen bis 38° erreicht. Bei der Durchführung der Messung müssen soweit wie möglich Fußbewegungen des Patienten vermieden werden, da diese zu Perfusionsveränderungen führen. Daher muß die vom Computer ermittelte Kurve eventuell unter Berücksichtigung von Bewegungsartefakten beurteilt werden. Diese Artefakte treten als Spitzenwerte auf und werden vom Computer ausgeschieden bzw. bei der Mittelwertbildung ignoriert. Nach Ermittlung der Kurve kann dann die Hyperthermielatenz von dieser Kurve gemessen werden.

Um den klinischen Hintergrund zu der Erfindung klarer darzustellen, wird nachfolgend das Ergebnis einer klinischen Untersuchung von 40 Personen beschrieben:
40 Personen wurden nach ihrer Klinik in 4 Untergruppen zugeteilt, die nachfolgend aufgeführt sind:
Gruppe 1 umfaßte 10 gesunde Probanden (5 männlich, 5 weiblich), die als Kontrollkollektiv dienten. In Gruppe 2 waren 10 Patienten (6 männlich, 4 weiblich) zusammengefaßt, welche seit mindestens 3 Jahren an einem oral behandelten Diabetes mellitus litten und häufige oder ständige Parästhesien verspürten, die jedoch vom neurologischen Status her völlig unauffällig waren. Wegen der bekannten Grunderkrankung und der typischen Anamnese mußte angenommen werden, daß diese Beschwerden den Beginn eines PNS darstellten ("burning feet syndrom"). Gruppe 3 rekrutierte sich aus weiteren 10 Probanden (7 männlich, 3 weiblich), welche ebenfalls seit mindestens 3 Jahren an einem Diabetes mellitus litten. Davon waren 9 mit oraler Antidiabetikamedikation eingestellt, 1 Proband war insulinpflichtig. Im Gegensatz zur Gruppe 2 hatten alle Patienten dieses Kollektivs an den unteren Extremitäten ausgeprägte Störungen im Bereich der Tiefen- und Oberflächensensibilität, die im neurologischen Status festgestellt werden konnten und jederzeit reproduzierbar waren. Um eine motorische Mitbeteiligung auszuschließen, wurde elektroneurographisch eine Messung der Nervenleitgeschwindigkeit und der distalen Latenz des Nervus peronaeus an der stärker betroffenen Extremität durchgeführt. Die Aufnahme in diese Gruppe erfolgte nur, wenn die Nervenleitgeschwindigkeit im Normbereich, das heißt über 41m/sec lag.
Als 4. Gruppe wurden 10 Probanden (8 männlich, 2 weiblich) zusammengefaßt, welche zusätzlich zur Sensibilitätsstörung auch motorische Ausfälle zeigten. Diese wurden elektroneurographisch anhand einer verlangsamten Nervenleitgeschwindigkeit des Nervus peronaeus ebenfalls an der stärker betroffenen unteren Extremität verifiziert. Von den Probanden erhielten 8 orale Antidiabetika, die restlichen 2 waren insulinpflichtig.

### Methodik:

Bei allen Probanden wurde die Mikrozirkulation der Haut an der stärker betroffenen unteren Extremität gemessen und über einen Zeitraum von 5 Minuten beobachtet. Gleichzeitig wurde mittels einer speziellen Sonde, die auf 44°C erwärmt war, die Hauttemperatur angehoben und die dabei zu beobachtende Mikrozirkulationszunahme mittels der zuvor beschriebenen Laserperfusionsmessung verfolgt.

Für die statistische Auswertung wurde die Zeit von Begann der Hyperthermie bis zum Auftreten einer Mikrozirkulationszunahme verwendet. Dieser Zeitraum wurde als sog. "Hyperthermielatenz" bezeichnet. Außerdem wurde der Ausgangsperfusionswert und der Maximalwert der Mikrozirkulation erfaßt.

Als Signifikanztest wurde einerseits das parameterfreie Verfahren nach Wilcoxon eingesetzt (siehe z.B. das Buch Guilford JP (1959) Fundamental Statistics in Psychology and Education. McGraw-Hill, New York: 587 - 588), andererseits wurde der ebenfalls in diesem Buch beschriebene Chi-Square-Test zur Überprüfung von Varianzunterschieden verwendet. Nach dem Wilcoxontest wurde die Signifikanzuntergrenze mit p≦0,02 festgelegt. Das Minimum für den Chi-Sguare-Test wurde mit P≦0,001 verankert.

### Ergebnisse:

40 Personen (26 männlich, 14 weiblich) wurden in die Untersuchung aufgenommen. Das Altersmaximum lag in allen Gruppen zwischen 55 und 70 Jahren bei einer relativ großen Streuung. Signifikante Unterschiede nach beiden verwendeten Tests waren nicht vorhanden.

Bei allen gesunden Probanden (Gruppe 1) stieg die Perfusion im Median nach 26 Sekunden. Der Median der Hyperthermielatenz lag in Gruppe 2 bei 64, in Gruppe 3 bei 180 und in Gruppe 4 bei 235 Sekunden. Die Gruppen 1 - 3 unterschieden sich im Wilcoxontest mit einer Signifikanz von p≦0,01, der Signifikanz zwischen sensiblen und motorischem PNS (Gruppe 3 und 4) betrug p≦0,02. Alle 4 Gruppen wiesen im Chi-Square-Test P-Werte von ≦0,0001 auf, womit eine sehr gute Differenzierung der Gruppenwerte möglich ist.

Die Ausgangsperfusionswerte überlappten sich in den untersuchten Kollektiven stark, was auch zu keiner Signifikanz nach dem Chi-Square-Test führte. Die besten diesbezüglichen Unterschiede waren zwischen Gruppe 1 und 4, also zwischen den zwei Extremgruppen erzielbar, jedoch war P mit einem Wert von 0,0016 noch über dem geforderten Limit von P≦0,001 des Chi-Square-Tests. Nach dem Wilcoxontest war der Unterschied in diesen beiden Gruppen mit p≦0,01 hochsignifikant, wodurch dokumentiert ist, daß die schwerste Form des PNS, die in dieser Studie vorkommt, in der Perfusionsverteilung deutlich unter der gesunden Kontrollgruppe liegt, daß jedoch eine Zuordnung des Einzelwertes in die jeweilige Gruppe statistisch nicht möglich ist.

Die maximalen Perfusionswerte zeigten ab Gruppe 3 erheblich niedrigere Werte. Beide Gruppen unterschieden sich von der Kontrollgruppe und von Gruppe 2 im Wilcoxontest mit p≦0,01 hochsignifikant, im Chi-Square-Test war P≦0,0001. Zwischen Gruppe 1 und 2 sowie 3 und 4 fanden sich weder im Wilcoxontest noch im Chi-Square-Test signifikante Maximalperfusionsunterschiede.

Eine weitere Differenzierung in männliche und weibliche Subgruppen brachte keine zusätzlichen Gesichtspunkte.

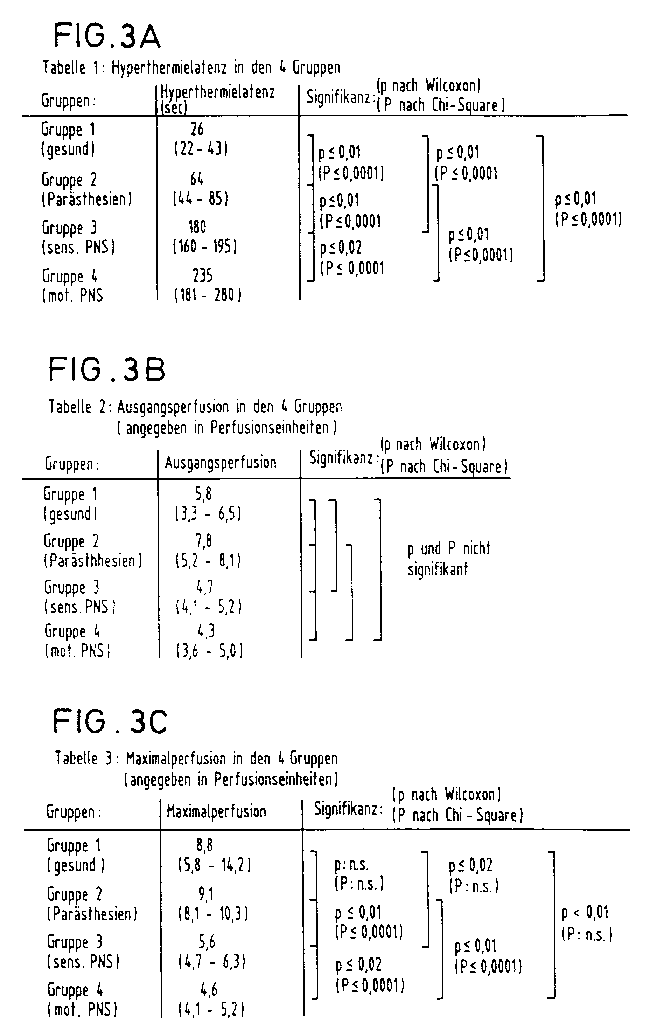

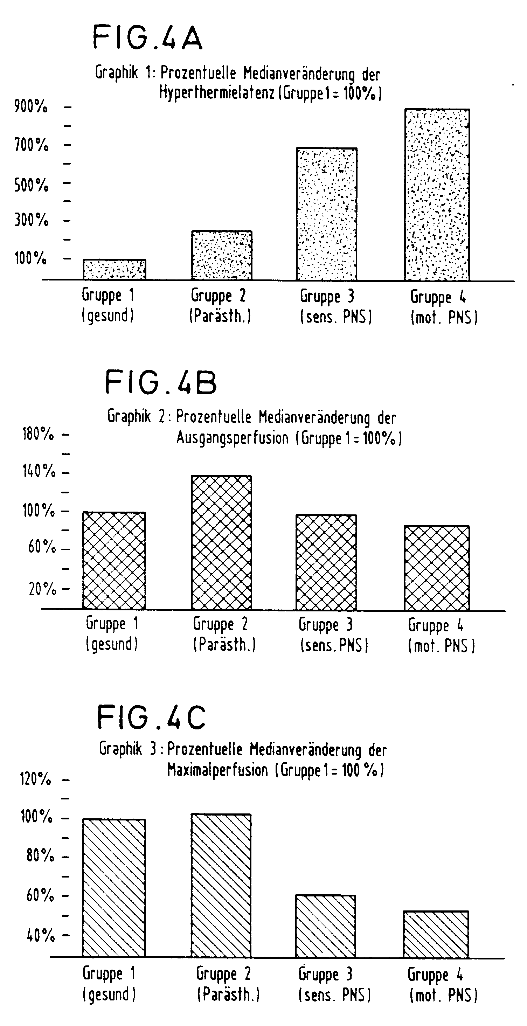

Die Tabellen 1 - 3 der Fig. 3a bis 3c stellen Hyperthermielatenz, Ausgangsperfusionswerte und Maximalperfusionswerte mit ihren Signifikanzen im Wilcoxontest sowie in Klammer stehend im Chi-Square-Test dar. Die Grafiken 1-3 der Fig. 4a bis 4c zeigen die prozentuellen Median-Unterschiede der drei Parameter.

### Diskussion:

Die Resultate dieser Untersuchung deuten klar darauf hin, daß die Provokation der Mikrozirkulation durch Hyperthermie Unterschiede in den Stadien des PNS-Syndroms zeigt. Vor allem die Hyperthermielatenz ist zur Differenzierung gut geeignet. Das Normalkollektiv kann mit Hyperthermielatenzwerten bis 50 Sekunden nach oben limitiert werden. Ein Vergleich der Einzeldaten der Gruppe 2 hat ergeben, daß 7 von den 10 Probanden mit Burning-Feet-Syndrom oberhalb dieses Wertes liegen, das bedeutet, daß bei 3 Probanden keine pathologisch verzögerne Hyperthermielatenz vorliegt. Unter der Voraussetzung, daß tatsächlich alle Probanden dieser Gruppe ihre Beschwerden im Rahmen eines incipienten PNS hatten, ergibt dies in diesem frühen Stadium eine Sensitivität von 70%. In den beiden Gruppen mit schweren polyneuropathischen Veränderungen befand sich kein einziger Proband mit seinen Hypethermielatenzwert unter 50 Sekunden.

Auffallend ist aber nicht nur die zunehmend verlängerte Hyperthermielatenz in den einzelnen Gruppen, sondern auch, daß die Mikrozirkulationszunahme in den beiden höchsten PNS-Gruppen bei weitgehend identischen Ausgangswerten deutlich abnimmt. Damit liegt die Überlegung nahe daß die Zunahme der Mikrozirkulation in Gruppe 3 und 4 überhaupt nicht als echter Anstieg zu werten sein könnte, sondern vielleicht lediglich eine durch die Hyperthermie forcierte stärkere Mikrozirkulationsschwankung darstellt. Aus einigen, derzeit noch nicht veröffentlichten Resultaten geht hervor, daß dies durchaus wahrscheinlich ist, manche Personen scheinen überhaupt erst nach 10 oder mehr Minuten die erforderliche Temperatur zu erreichen, welche eine deutliche Mikrozirkulationszunahme induziert. Nach 5-minütiger Hyperthermie mit einer 44 Grad Sonde werden maximal Hauttemperaturen von 36° erzielt, die weitere Temperaturzunahme geschieht dann sehr langsam.

Sicherlich muß man bedenken, daß die Stimulation der Mikrozirkulation eine Leistung des autonomen Nervensystems darsrellt, aber die Differenzen der Hyperthermielatenz sind in den Gruppen eindeutig und die Häufigkeit der autonomen Neuropathie ist im Zusammenhang mit motorischen und sensiblen Ausfällen bei Polyneuropathiesyndromen oftmals untersucht und beschrieben worden, genauer gesagt ist eine Trennung in einzelne unabhängige klinische Neuropathieformen praktisch unmöglich, dies zeigen auch die häufigen Klassifikationsversuche dieser Erkrankung.

Sicher wird die hypertherme Laser-Doppler-Flowmetrie die herkömmliche Elektroneurographie bei der Diagnose des motorischen PNS nicht verdrängen können und dies ist auch nicht das Ziel dieser Studie gewesen, sondern diese Untersuchungstechnik macht es erstmals möglich, eine routinemäßige Diagnostik und Differenzierung der einzelnen Stadien eines PNS durchzuführen und damit unter Umständen auch einen möglichen Therapieerfolg objektivieren zu können. Die Diagnose eines PNS ist mit Hilfe der hyperthermen Laser-Doppler-Flowmetrie zu einem Zeitpunkt möglich, wo eine Behandlung noch bessere Erfolgschancen hat, als in späteren Stadien.

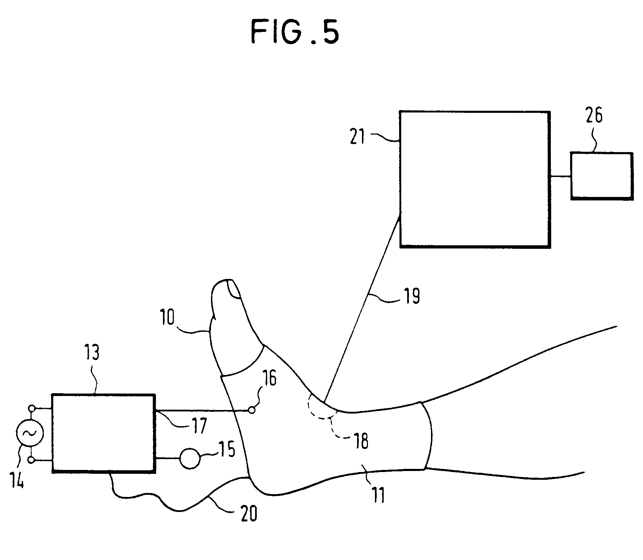

Die Fig. 5 zeigt eine alternative erfindungsgemäße Vorrichtung, bei der statt Perfusionswerten Temperaturwerte gemessen werden.

Die Vorrichtung ist in manchen Hinsichten der Vorrichtung gemäß Fig. 1 sehr ähnlich, weshalb für die gleichen Teile die gleichen Bezugszeichen verwendet worden sind. Insbesondere ist die Regelschaltung für die Heizdecke mit der in Fig. 1 identisch.

Im Prinzip ist die Meßanordnung sehr ähnlich, die Lasersonde 23 sowie das Laserperfusionsmeßgerät 24 sind jedoch nicht vorhanden und die verwendete Temperaturmeßsonde ist gegenüber der Heizdecke 11, die sie umgibt, isoliert. Die von der Temperatursonde 18 ermittelte Hauttemperatur wird über die Leitung 19 wie bisher an einen Mikrocomputer 21 angelegt, welcher den zeitlichen Verlauf der Hauttemperatur aufnimmt und speichert und die Anstiegszeiten zwischen bestimmten Temperaturen aus dem ermittelten und gespeicherten Verlauf ermittelt.

Mit anderen Worten wird hier die Zeit gemessen, in welcher bei aufgeheizter Heizdecke die Hauttemperatur ansteigt. Vor allem bieten sich hier Temperaturänderungen in 1 bis 2°-Schritten ab 30°C aufwärts bis maximal etwa 36°C an.

Bei leichten neuropathischen Veränderungen ist erfindungsgemäß festgestellt worden, daß die Zeitspanne des Hauttemperaturanstieges von 34 auf 36° gegenübergesunden Probanden verlängert ist. Bei schwereren polyneuropathischen Veränderungen tritt auch eine Verlängerung der Zeitspanne zwischen 32 und 34° dazu, und bei noch schwereren Formen der Polyneuropathie wird auch die Zeitspanne von 30 bis 32° erhöht.

Bei der in den Versuchen verwendeten Heizecke, welche eine gewisse "Vorlaufzeit" hat, bis sie die 44° voll erreich, liegen die Normbereiche, d.h. bei gesunden Probanden, für 30 bis 32° bis 50 Sekunden, für 32 bis 34° bis 30 Sekunden und für 34 bis 36° bis 25 Sekunden.

Schwere Stadien erreichen oft 36°C in der Meßzeit von bis zu 5 Minuten gar nicht und haben z.B. bei 30 bis 32° eine "Hauttemperaturlatenz" von 100 Sekunden und mehr.

Insgesamt sind bislang Messungen an etwa 100 Patienten durchgeführt worden und die Korrelationen zeigen sich mit der hypothermen Laser-Doppel-Flowmetrie, vor allem ab 32° aufwärts. Wenn darunter auch Hauttemperaturverzögerungen in massivem Ausmaß auftreten, ist in der hypothermen Laser-Doppler-Flowmetrie überhaupt kein Anstieg mehr erzielbar. Somit kann man sagen, daß die Methode mit der Laserperfusionsmessung zwar eine frühere, aber auch störanfälligere Messung ist. Muskelverspannungen sind trotz guter Software nicht auszuschalten und gehen eher ein, bei der Temperaturmessung spielen sie kaum eine Rolle.

In schweren Stadien ist eine bessere Beurteilung durch die Temperaturmethode gegeben, da hier mit der hypothermen Laser-Doppler-Flowmetrie überhaupt kein Anstieg und somit auch keine Hyperthermielatenz gemessen werden kann. Dies ist vor allem für Medikamentenstudien wichtig.

Schließlich soll erwähnt werden, daß auch bei der Ausführung gemäß Fig. 5 der Computer in der Lage ist, für jeden Probanden ein Protokoll in Form einer Computergraphik zu erstellen, woraus Vergleiche zwischen einem bestimmten Probanden und einem Normprobanden bzw. für einen einzigen Probanden Vergleiche zwischen den ermittelten Werten vor und nach einer Behandlung darstellbar sind, wobei auch diese Computergraphik über den Drucker 26 ausgedruckt werden kann.

Die Vorrichtung gemäß Fig. 5 hat den Vorteil, daß sie relativ preisgünstig hergestellt werden kann, da nicht unbedingt ein relativ teures Perfusionsmeßgerät erforderlich ist.

## Patentansprüche

1. Verfahren zur Feststellung, ob bei einem Probanden eine Nervenschädigung (Polyneuropathie) vorhanden ist bzw. des Grades der Nervenschädigung, dadurch gekennzeichnet, daß an einer Extremitat (10) des Probanden eine Erhöhung der Hauttemperatur verursacht wird, daß gleichzeitig der Perfusionswert oder ein mit diesem Perfusionswert korrelierter Wert, an dieser Extremität (10) gemessen wird, und daß die Zeit vom Einsetzen der Hauttemperaturzunahme bis zum Einsetzen einer Perfusionszunahme aufgrund der erhöhten Hauttemperatur als Maß für das Vorhandensein bzw. den Grad der Nervenbeschädigung Hermittelt wird.

2. Verfahren nach Anspruch 1, dadurch gekennzeichnet, daß die Messung der Perfusion und die Erhöhung der Hauttemperatur des Probanden am Fuß vorzugsweise im Knöchel-Bereich vorgenommen wird.

3. Verfahren nach Anspruch 1 oder 2, dadurch gekennzeichnet daß die Erhöhung der Temperatur mit einer Heizdecke (11) vorgenommen wird, welche auf eine Temperatur im Bereich vom 42 bis 46°, vorzugsweise von 44° beheizt wird, daß vorzugsweise die Ermittlung der Zeit aufgrund einer Perfusionszunahme von bis zu 25%, vorzugsweise von 10% über den Ausgangs-Mittelwert vorgenommen wird, wobei insbesondere die Ermittlung der Zeit aufgrund einer Temperaturzunahme von ca. 1° über den Ausgangs-Mittelwert vorgenommen wird.

4. Vorrichtung zur Feststellung, ob bei einem Probanden eine Nervenschädigung (Polyneuropathie) vorhanden ist bzw. des Grades der Nervenschädigung, insbesondere zur Durchführung des Verfahrens nach einem der vorhergehenden Ansprüche, gekennzeichnet durch eine Heizvorrichtung (11) zur Erhöhung der Hauttemperatur einer Extremitat (10) des Probanden, durch eine Einrichtung (18) zur Ermittlung der Hauttemperatur an der Extremität (10) des Probanden, durch eine Einrichtung (24) zur Messung der Perfusionswerte an der Extremität (10), und durch eine Einrichtung (21) zur Feststellung des Zeitintervalls von einer eindeutigen Hauttemperaturzunahme bis zum Einsetzen einer eindeutigen Perfusionszunahme.

5. Vorrichtung nach Anspruch 4, dadurch gekennzeichnet, daß die Heizvorrichtung (11) eine Heizdecke ist, wobei vorzugsweise eine Regelschaltung (13) vorgesehen ist, um die Temperatur der Heizdecke auf einen erwünschten Wert hinzuregeln, und daß die Einrichtung (24) zur Ermittlung der Perfusionswerte vorzugsweise eine Laserperfusionsmessung durchführt.

6. Vorrichtung nach einem der vorhergehenden Ansprüche 4 oder 5, dadurch gekennzeichnet, daß die Einrichtung (18) zur Ermittlung der Hauttemperatur vorzugsweise eine in Kontakt mit der Haut des Probanden bringbare Sonde ist, und vorzugsweise daß die Temperatursonde einen Meßkopf mit einem zumindest im wesentlichen mittig angeordneten Loch aufweist, wobei die für die Perfusionsmessung erforderliche Lasersonde (23) im genannten Loch plazierbar ist, wobei die Temperatursonde (18) insbesondere in der Heizdecke eingebaut ist.

7. Vorrichtung nach einem der vorhergehenden Ansprüche, dadurch gekennzeichnet, daß ein Computer (21) oder Mikroprozessor vorgesehen ist, welcher die Temperaturwerte und die Perfusionswerte aufnimmt und hieraus das Zeitintervall von der Hauttemperaturzunahme bis zur Perfusionszunahme ermittelt, und vorzugsweise ein Protokoll zu der durchgeführten Messung liefert.

8. Verfahren zur Feststellung, ob bei einem Probanden eine Nervenschädigung (Polyneuropathie) vorhanden ist bzw. des Grades der Nervenschädigung, dadurch gekennzeichnet, daß an einer Extremität (10) des Probanden eine Erhöhung der Hauttemperatur mittels einer Heizvorrichtung (11) verursacht wird, daß an einem im Bereich dieser Extremität gelegenen Stelle, die gegen direkte Beheizung durch die Heizvorrichtung (11) isoliert ist, die Hauttemperatur gemessen wird und daß die Anstiegzeiten der an der isolierten Stelle gemessenen Hauttemperatur in einem oder mehreren Temperaturbereich(en) für die Feststellung ermittelt werden.

9. Verfahren nach Anspruch 8, dadurch gekennzeichnet, daß die Messung der Anstiegzeiten erst dann vorgenommen wird, wenn die Heizvorrichtung (11) die vorgegebene Solltemperatur, beispielsweise 44°C, erreicht hat, und insbesondere daß die Anstiegzeiten im Temperaturbereich von 30°C bis 36°C, beispielsweise in den Bereichen 30 bis 32°C, 32 bis 34°C und 34 bis 36°C gemessen werden.

10. Vorrichtung zur Feststellung, ob bei einem Probanden eine Nervenschädigung (Polyneuropathie) vorhanden ist bzw. des Grades der Nervenschädigung, insbesondere zur Durchführung des Verfahrens nach einem der Ansprüche 8 oder 9, gekennzeichnet durch eine Heizvorrichtung (11) zur Erhöhung der Hauttemperatur einer Extremität (10) des Probanden, durch einen von der Heizvorrichtung (11) isolierten Hauttemperaturfühler (18), sowie durch eine Einrichtung (21) zur Messung der zeitlichen Zunahme der Hauttemperatur und zur Ermittlung der Anstiegzeiten zwischen ausgewählten Hauttemperaturen und vorzugsweise durch einen Mikrocomputer zur Aufnahme des zeitlichen Verlaufs der Hauttemperatur und zur Ermittlung der Anstiegzeiten aus dem ermittelten Verlauf sowie ggf. zum Vergleich der ermittelten Anstiegszeiten mit bekannten Anstiegszeiten vom gleichen Probanden oder mit vorher ermittelten Normwerten.

## Claims

1. Method of determining whether a subject has nerve damage (polyneuropathy) and/or the degree of the nerve damage, characterized in that an increase of the skin temperature is provoked at an extremity (10) of the subject, that at the same time the perfusion value or a value correlated with this perfusion value is measured at this extremity (10), and the time from the start of the increase in temperature of the skin up to the start of an increase in perfusion as a result of the elevated skin temperature is determined as a measure for the presence and/or the degree of the nerve damage.

2. Method in accordance with claim 1, characterized in that the measurement of the perfusion and the increase of the skin temperature of the subject is effected at the foot, preferably in the ankle region.

3. Method in accordance with claim 1 or claim 2, characterized in that the increase of the temperature is effected with a heated blanket (11) which is heated to a temperature in the range from 42 to 46°, preferably 44°C, in that the determination of the time is preferably effected as a result of an increase in perfusion of up to 25 %, preferably of 10 % above the initial mean value, with the determination of time being effected in particular as a result of an increase in temperature of ca. 1°C above the critical mean value.

4. Apparatus for determining whether a nerve damage (polyneuropathy) is present in a subject, and/or the degree of the nerve damage, in particular for carrying out the method of one of the preceding claims, characterized by a heating device (11) for increasing the skin temperature of an extremity (10) of the subject, by means (18) for determining the skin temperature at the extremity (10) of the subject, by means (24) for measuring the perfusion values at the extremity (10), and by means (21) for determining the time interval from a clear increase in skin temperature up to the start of a clear increase in perfusion.

5. Apparatus in accordance with claim 4, characterized in that the heating device (11) is a heating blanket; in that a control circuit (13) is preferably provided in order to regulate the temperature of the heated blanket to a desired value; and in that the means (24) for determining the perfusion values carries out a laser perfusion measurement.

6. Apparatus in accordance with one of the preceding claims 4 or 5, characterized in that the means (18) for determining the skin temperature is a probe which can be brought into contact with the skin of the subject, and preferably in that the temperature probe has a measuring head with an at least substantially centrally arranged hole, with the laser probe (23) required for the perfusion measurement being placeable in the said hole, and with the temperature probe (18) being in particular built into the heated blanket.

7. Apparatus in accordance with one of the preceding claims, characterized in that a computer (21) or microprocessor is provided which records the temperature values and the perfusion values and determines the time interval from the increase in skin temperature up to the increase in perfusion from these values, and which preferably delivers a protocol of the measurement which has been effected.

8. Method of determining whether a subject has nerve damage (polyneuropathy) and/or the degree of the nerve damage, characterized in that an increase of the skin temperature is provoked at an extremity (10) of the subject by a heating device (11); in that the skin temperature is measured in the region of this extremity at a position which is insulated against direct heating by the heating device (11); and in that the rise times of the skin temperature measured at the insulated position is determined in one or more temperature ranges for the determination.

9. Method in accordance with claim 8, characterized in that the measurement of the rise times is first effected when the heating device (11) has reached a predetermined desired temperature, for example 44°, and in particular that the rise times are measured in the temperature range from 30°C to 36°C, for example in the ranges 30 to 32°C, 32 to 34°C and 34 to 36°C.

10. Apparatus for determining whether a subject has nerve damage (polyneuropathy) and/or the degree of the nerve damage, in particular for carrying out the method of one of the claims 8 or 9, characterized by a heating device (11) for increasing the skin temperature of an extremity (10) of the subject, by a skin temperature sensor (18) insulated from the heating device (11), and also by a means (21) for measuring the time increase of the skin temperature and for determining the rise times between selected skin temperatures, and preferably by a microcomputer for recording the temporal variation of the skin temperature and for determining the rise times from the temporal variation which is found, and also optionally for comparing the rise times that are determined with known rise times from the same subject or with previously derived normed values.

## Revendications

1. Procédé pour déterminer si un sujet présente des destructions neurologiques (polyneuropathies) et/ou le degré de destruction neurologique, caractérisé en ce que l'on provoque une augmentation de la température cutanée à une extrémité (10) du sujet, en ce que l'on mesure simultanément à cette extrémité (10) la valeur de perfusion ou une valeur corrélée avec cette valeur de perfusion, et en ce que l'on détermine le temps depuis le commencement de l'augmentation de température de la peau jusqu'au commencement d'une augmentation de la perfusion qui résulte de l'augmentation de température cutanée, en tant que mesure pour la présence et/ou le degré de destruction neurologique.

2. Procédé selon la revendication 1, caractérisé en ce que l'on effectue la mesure la perfusion et l'augmentation de la température cutanée du sujet au niveau du pied, de préférence dans la région de la cheville.

3. Procédé selon l'une ou l'autre des revendications 1 et 2, caractérisé en ce que l'on effectue l'augmentation de la température au moyen d'une couverture chauffante (11) qui est chauffée à une température dans la plage de 42 à 46°, de préférence 44°C, en ce que l'on effectue de préférence la détermination du temps lorsque l'augmentation résultante de la perfusion s'élève jusqu'à 25%, de préférence 10% au-dessus de la valeur moyenne initiale, la détermination du temps étant effectuée en particulier en résultat d'une augmentation de température d'environ 1°C au-dessus de la valeur initiale moyenne.

4. Appareil pour déterminer si un dérangement neurologique (polyneuropathie) est présent dans un sujet, et/ou le degré du dérangement neurologique, en particulier pour mettre en oeuvre le procédé de l'une des revendications précédentes, caractérisé en ce qu'il comprend un dispositif chauffant (11) pour augmenter la température cutanée d'une extrémité (10) du sujet, des moyens (18) pour déterminer la température cutanée à l'extrémité (10) du sujet, des moyens (24) pour mesurer les valeurs de perfusion à l'extrémité (10), et des moyens (21) pour déterminer l'intervalle de temps depuis une augmentation franche de la température cutanée jusqu'au commencement d'une augmentation franche de la perfusion.

5. Appareil selon la revendication 4, caractérisé en ce que le dispositif chauffant (11) est une couverture chauffante; en ce qu'il est de préférence prévu un circuit de commande (13), afin de réguler la température de la couverture chauffante à une valeur désirée; et en ce que les moyens (24) pour déterminer les valeurs de perfusion procèdent à une mesure de perfusion par laser.

6. Appareil selon l'une des revendications précédentes 4 ou 5, caractérisé en ce que les moyens (18) pour déterminer la température cutanée sont une sonde qui peut être mise en contact avec la peau du sujet et de préférence en ce que la sonde de température comporte une tête de mesure avec un trou agencé au moins sensiblement au centre, la sonde laser (23) nécessaire pour la mesure de perfusion pouvant être placée dans ledit trou, et la sonde de température (18) étant en particulier intégrée à la couverture chauffante.

7. Appareil selon l'une quelconque des revendications précédentes, caractérisé en ce qu'il est prévu un ordinateur (21) ou microprocesseur, qui enregistre les valeurs de température et les valeurs de perfusion et détermine à partir de ces valeurs l'intervalle de temps depuis l'augmentation de la température cutanée jusqu'à l'augmentation de perfusion, et qui fournit de préférence un compte-rendu de la mesure qui a été effectuée.

8. Procédé pour déterminer si un sujet présente un dommage neurologique (polyneuropathie) et/ou le degré du dommage neurologique, caractérisé en ce que l'on provoque une augmentation de la température cutanée à une extrémité (10) du sujet au moyen d'un dispositif chauffant (11); en ce que l'on mesure la température cutanée dans la région de cette extrémité à un emplacement qui est isolé contre un chauffage direct par le dispositif chauffant (11); et ce que l'on calcule le temps de montée de la température cutanée mesurée à l'emplacement isolé, pour une ou plusieurs plages de température pour la détermination.

9. Procédé selon la revendication 8, caractérisé en ce que l'on effectue la mesure du temps d'augmentation uniquement lorsque le dispositif chauffant (11) a atteint une température désirée prédéterminée, par exemple 44°C, et en particulier en ce que l'on mesure le temps d'augmentation dans la plage de température de 30 à 36°C, par exemple dans les plages de 30 à 32°C, 32 à 34°C et 34 à 36°C.

10. Appareil pour déterminer si un sujet présente un dommage neurologique (polyneuropathie) et/ou le degré du dommage neurologique, en particulier pour mettre en oeuvre le procédé selon l'une des revendications 8 et 9, caractérisé en qu'il comprend un dispositif chauffant (11) destiné à augmenter la température cutanée d'une extrémité (10) du sujet, par un détecteur de température cutanée (18) isolé du dispositif chauffant (11) ainsi que des moyens (21) pour mesurer le temps d'augmentation de la température cutanée et pour déterminer le temps d'augmentation entre des températures cutanées choisies et de préférence un microordinateur pour enregistrer les variations temporelles de la température cutanée et pour déterminer les temps d'augmentation à partir des variations temporelles qui est détectée, et en option pour comparer les temps d'augmentation déterminés avec des temps d'augmentation connus du même sujet ou avec des valeurs standard préalablement déterminées.
